# EUROPÄISCHE PATENTSCHRIFT

(11) **EP 2 172 580 B1**
(45) Veröffentlichungstag und Bekanntmachung des Hinweises auf die Patenterteilung: **06.11.2019**
(21) Anmeldenummer: 09170896.6
(22) Anmeldetag: 22.09.2009
(51) Int. Cl.: A61L 31/02, A61L 31/08, A61L 31/10, A61L 31/14, A61L 31/16, C25D 9/10, C25D 11/36, A61F 2/82

(54) **Implantat und Verfahren zur Herstellung desselben**
Implant and method for manufacturing same
Implant et methode de fabrication dudit implant

(30) Priorität: 06.10.2008 DE 102008042602; 29.04.2009 DE 102009002709
(43) Veröffentlichungstag der Anmeldung: 07.04.2010
(73) Patentinhaber: BIOTRONIK AG, 8180 Bülach (CH)
(72) Erfinder: Bayer, Ullrich, 18211 Admannshagen-Bargeshagen (DE); Becher, Bärbel, 18057 Rostock (DE); Block, Bernd, 18055 Rostock (DE); Vögele, Robert, 18055 Rostock (DE); Decker, Patricia, 18258 Schwaan (DE)
(74) Vertreter: Keck, Hans-Georg

(56) Entgegenhaltungen:
- WO-A1-92/13984
- WO-A1-2009/046532
- WO-A2-2008/051680
- US-A- 4 704 126
- US-A- 5 310 464
- US-A- 5 354 390
- US-B1- 6 679 980

## Beschreibung

Die vorliegende Erfindung betrifft ein Verfahren zur Herstellung eines Implantats, insbesondere einer intraluminalen Endoprothese, mit einem Körper, der überwiegend Eisen aufweist, insbesondere eine Eisenlegierung, sowie ein entsprechendes Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heute werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Eisen, Mangan, Zink und/oder Wolfram, insbesondere eine Eisenbasislegierung (im Folgenden kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat nach einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. zum Beispiel auf 80% des Nominalwerts, abgefallen ist. Das Implantat kann somit bei vorhandener Integrität seiner Hauptfunktion, die Durchlässigkeit des Gefäßes zu gewährleisten, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung zum Beispiel 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d.h. erst nach einer Verweildauer in dem behandelten Organismus von ca. 2 Jahren. Dies bedeutet, dass sich der Kollapsdruck bei eisenhaltigen Implantaten für diese Anwendung über die Zeit zu langsam verringert.

Im Stand der Technik sind bereits unterschiedliche Mechanismen der Degradationskontrolle von Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem degradablen Grundkörpermaterial (z.B. Magnesium oder Mg-Legierungen) abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemischen Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

In US 6679980 B1 sind zudem elektropolierte Stents beschrieben. In US 5354390 werden Implantate aus Titan oder Titanlegierungen offenbart. In US 4704126 werden chemisch polierte Implantate beschrieben. In WO 2008/051680 A2 werden Stents aus Magnesium offenbart. In US 5310464 wird ein Prozess zur Bereitstellung von Phosphatbeschichtungen auf Prothesen offenbart. In WO 92/13984 wird ein Prozess zur Bereitstellung von bioaktiven Beschichtungen beschrieben. In WO 2009/046532 A1 werden medizinische Geräte mit einer Calciumphosphatbeschichtung offenbart.

Folglich besteht die Aufgabe der vorliegenden Erfindung darin, ein Verfahren zur Herstellung eines Implantats anzugeben, das eine Degradation des Implantats im gewünschten Zielkorridor bewirkt, insbesondere bei Implantaten mit einer Eisenbasislegierung in einem kürzeren Zeitraum. Dabei soll die Degradation zu einem kontrollierbaren Zeitpunkt stattfinden. Entsprechend besteht die Aufgabe der Erfindung außerdem darin, ein derartiges Implantat zu schaffen.

Die obige Aufgabenstellung wird gelöst durch ein Verfahren umfassend die folgenden Schritte:
a) Bereitstellen des Körpers des Implantats, der überwiegend Eisen aufweist,
b) Einlagerung von Wasserstoff in zumindest einen Teil des oberflächennahen Gefüges des Implantatkörpers durch Beizen mittels einer Säure, wobei im Anschluss an den Beizschritt zur Einlagerung des Wasserstoffs zumindest ein Teil des Implantatkörpers in ein saures bis basisches Elektrolytsystem getaucht und anschließend mit der Kathode einer Spannungsquelle verbunden wird.

Bei der vorliegenden Erfindung umfasst der Körper des Implantats mindestens einen Teil des Implantats, vorzugsweise den Hauptteil des Implantats, welcher die mechanische Integrität des Implantats bewirkt.

Unter dem Begriff "Gefüge" wird im Folgenden die Anordnung der Bestandteile von Feststoffen (Festkörpern), insbesondere die Anordnung der Kristallite (Körner), Poren, amorphen Bereiche und Korngrenzbereiche des Implantatkörpers verstanden. Ferner wird unter dem Ausdruck "oberflächennahes Gefüge" der Volumenbereich des Gefüges des Implantatkörpers verstanden, welcher sich von der Oberfläche bis in eine bestimmte (geringe) Tiefe des Implantatkörpers erstreckt. Dieser sich von der Oberfläche bis in eine bestimmte (geringe) Tiefe erstreckende Volumenbereich des Implantatkörpers wird auch als "oberflächennah angeordnete Grenzschicht des Implantatkörpers" oder kurz als "Grenzschicht" bezeichnet.

Der Vorteil des erfindungsgemäßen Verfahrens besteht darin, dass durch die Einlagerung des Wasserstoffs, vorzugsweise von atomarem Wasserstoff, eine Versprödung des Gefüges erfolgt, welche sich in einer Erhöhung der Festigkeit des oberflächennahen Gefüges und einer damit verbundenen Verschlechterung der Dehnungseigenschaften dieses Gefügebereichs äußert. Hierdurch wird, abhängig von der vorliegenden mittleren Korngröße des Implantatkörper-Materials, eine lokale Werkstoffschädigung in dem oberflächennahen Bereich des Implantatkörpers erzielt. Die versprödende Wirkung des Wasserstoffs führt zu einer Beschleunigung der Degradation in diesem Bereich, insbesondere aufgrund des beschleunigten Herauslösens der Kristallite aus dem Gefügeverbund. Das beschleunigte Herauslösen der Kristallite wird vor allem dadurch bewirkt, dass in der Regel an den Korngrenzen die höchste Wasserstoff-Konzentration vorliegt. Die heraus gelösten Körner werden durch die umgebende Körperflüssigkeit von der Oberfläche des Implantats entfernt sowie bevorzugt korrodiert oder aufgelöst. Die verbleibende Oberfläche des Implantats wird durch das Herauslösen der Kristallite aufgeraut bzw. mit einer zerklüfteten Struktur versehen. In der Folge entsteht ein erhöhter Oberflächeninhalt, welcher zu einer weiteren Beschleunigung der Degradation des Implantats beiträgt.

Da die mit Wasserstoff angereicherte Grenzschicht eine höhere Defektdichte als die darunter liegenden Gefügebereiche aufweist, zeigen die oberflächennahen Gefügebereiche eine geringere Bruchdehnung. Die durch die höhere Defektdichte entstehenden Risse laufen von der Randzone in die Richtung des nicht wasserstoffbeladenen Implantatkörpers hinein. Dort werden diese durch das hohe Rissenergieaufnahmevermögen des Grundwerkstoffs gestoppt.

In einem bevorzugten Ausführungsbeispiel erfolgt die Einlagerung von Wasserstoff in das Gefüge einer oberflächennah angeordneten Grenzschicht des Implantatkörpers, wobei die Grenzschicht eine Dicke von maximal 15 µm aufweist.

Die oberflächennah angeordnete Grenzschicht, die eine erhöhte Konzentration an Wasserstoff aufweist, muss sich ausgehend von der Oberfläche bis in eine für die jeweilige Anwendung angegebene optimale Tiefe erstrecken, da bei einer Wasserstoffbeladung in einem zu ausgedehnten Volumenbereich die Gefahr besteht, dass das Implantat bei oder nach einer mechanischen Beanspruchung, bspw. beim Dilatieren eines Stents, durch einen verzögerten Sprödbruch bricht. Die Beladungstiefe wird durch den Umformgrad des metallischen Materials, vorzugsweise der Eisenlegierung, den Rekristallisationszustand und die hierdurch erzeugte mittlere Korngröße, das Verfahren, mittels dem die Einlagerung des Wasserstoffs erfolgt, die Zusammensetzung der beteiligten Reagenzien sowie die Zusammensetzung des oberflächennahen Volumens des Implantatkörpers bestimmt.

Die mittlere Konzentration von Wasserstoff in den Gefügebereichen des Implantatkörpers, in denen die Einlagerung erfolgt, nach Abschluss der Einlagerung etwa 50 ppm bis etwa 150 ppm. Zum Vergleich: Die Gefügebereiche des Implantats, welche nicht zusätzlich mit Wasserstoff beladen wurden, weisen einen maximalen Wasserstoff-Gehalt von 15 ppm auf.

Eine Konzentration von Wasserstoff in den jeweiligen oberflächennahen Gefügebereichen in dem angegebenen Konzentrationsbereich bewirkt zum einen, da die Wasserstoffkonzentration nicht zu hoch gewählt wurde, dass die Oberflächenbereiche sich nicht sofort und unmittelbar vom Implantat lösen, und zum anderen, dass die Degradation beschleunigt erfolgt. Es kann jedoch auch schon bei geringeren Wasserstoffkonzentrationswerten, beispielsweise im Bereich von 30 ppm, eine Beschleunigung der Degradation insbesondere dann erreicht werden, wenn der behandelte Teil des Implantatkörpers einen erhöhten Umformgrad aufweist.

Ein besonders einfaches und kostengünstiges Verfahren, um eine Einlagerung von Wasserstoff in einen Teil des oberflächennahen Gefüges des Implantatkörpers zu erreichen, besteht darin, dass zumindest ein Teil des Implantatkörpers mittels einer anorganischen Säure gebeizt und anschließend in destilliertem Wasser gespült wird, wobei im Anschluss an den Beizschritt zur Einlagerung des Wasserstoffs zumindest ein Teil des Implantatkörpers in ein saures bis basisches Elektrolytsystem getaucht und anschließend mit der Kathode einer Spannungsquelle verbunden wird. Besonders bevorzugt werden zum Beizen die Säuren HCl und/oder HNO₃ verwendet. Ein anschließendes Spülen in destilliertem Wasser bewirkt ein Stopp des Beizprozesses und somit ein Stopp der weiteren Wasserstoffeinlagerung in das Gefüge des Implantatkörpers.

Außerdem kann die durch das Beizen aufgeraute Struktur der Körperoberfläche als Stoffreservoir für eine unten näher beschriebene weitere Beschichtung mittels einer pharmazeutisch aktiven Substanz dienen, welche als Nano- oder Mikropartikel eingelagert wird und beispielsweise das Knochenwachstum fördernde Substanzen wie Kalziumphosphate, temporär wirkende Kontrastmittel und/oder zellwachstumshemmende und/oder radioaktive Substanzen umfassen kann. Ferner kann in die aufgeraute Struktur effektiv Gleitmittel zur Verringerung des Reibungskoeffizienten in einem Katheter eingelagert werden.

Wesentliche Verfahrensparameter des Beizverfahrens, mittels denen die Degradationseigenschaften eingestellt werden können, sind die Zusammensetzung der Beize, die Beiztemperatur und die Beizzeit. Ferner spielen Materialparameter des Implantatkörpers eine Rolle, insbesondere die Zusammensetzung des Materials, der Verformungszustand, die Korngröße und die Zusammensetzung des Materials an der Oberfläche bzw. in unmittelbarer Nähe der Oberfläche.

In einem Ausführungsbeispiel wird zur Einlagerung des Wasserstoffs zumindest ein Teil des Implantatkörpers in ein saures bis basisches Elektrolytsystem getaucht und anschließend mit der Kathode einer Spannungsquelle verbunden, die mit einer Stromdichte von etwa 0,5 A/dm² bis etwa 2 A/dm² beaufschlagt wird. Hierfür wird der Implantatkörper elektrisch mit Anschlussdrähten, die bspw. Titan enthalten, verbunden. Im Elektrolytbehälter befindet sich eine Gegenelektrode aus einem rost- und säurebeständigen Stahl.

Nach dem Eintauchen des Implantats in den Elektrolyt wird das Implantat kathodisch geschaltet und mit der angegebenen Stromdichte beaufschlagt. Die an der Grenzfläche des wässerigen Elektrolyten zur Implantatoberfläche ablaufenden Effekte bewirken eine verstärkte Dissoziierung des wässerigen Elektrolyten, welche mit einer verstärkten Wasserstoffbildung einhergeht. Aufgrund der kathodischen Schaltung beginnt ein großer Anteil des Wasserstoffs in das Implantat hinein zu diffundieren. Abhängig von dem durch die Verfahrensparameter einstellbaren Diffusionsstrom des Wasserstoffs im Implantatkörpermaterial, von dem angelegten Potential, von der Behandlungszeit und von dem Gefügezustand des Materials des Implantatkörpers, der bspw. aus der Verformung der Legierung resultiert, der Korngröße und die Zusammensetzung des Materials an der Oberfläche bzw. in unmittelbarer Nähe der Oberfläche, kommt es zu einer hinsichtlich der Geschwindigkeit und der Eindringtiefe unterschiedlich verlaufenden Inkorporation von Wasserstoff in die oberflächennahen Gefügebereiche des Implantatkörpers, welche die erzielte Degradationsdauer bestimmt. Weiterhin ist die Einlagerung auch abhängig von der Gitterstruktur des Materials des Implantatkörpers. Beispielsweise ist der Wasserstoffdiffusionskoeffizient in einer Eisenlegierung mit einem 20 Gew.% Anteil von Mn mit einem kubisch-flächenzentrierten Gitter kleiner als bei dem kubisch-raumzentrierten Gitter von Reineisen. Als thermodynamisch bevorzugte Diffusionswege des Wasserstoffs dienen insbesondere die Korngrenzen. Die Degradationseigenschaften des behandelten Implantats hängen außerdem von der Zusammensetzung des galvanischen Elektrolyten und den elektrischen Parametern ab. Auch die Wasserstoffbeladung des Implantatkörpers wird von einer Aufrauung der Oberfläche des Implantats begleitet.

Ein weiterer Vorteil des erfindungsgemäßen Verfahrens nach dem obigen Ausführungsbeispiel besteht darin, dass die kathodisch gestützte Oberflächenbehandlung lediglich eine wenig kostenintensive Anlagentechnik erfordert. Die Oberflächenbehandlung kann beispielsweise in galvanischen Anlagen für die Goldabscheidung durchgeführt werden. Eine solche muss für die kathodisch gestützte Oberflächenbehandlung lediglich einen säurefesten Behälter und Gegenelektroden aus chemisch resistentem Materialien, z.B. ein Lochblech aus platiniertem Titan, aufweisen.

Vorzugsweise weist das wässrige Elektrolytsystem einen Gehalt an 85%-iger Phosphorsäure im Bereich zwischen 20 und 30 Volumen% auf.

In einem weiteren bevorzugten Ausführungsbeispiel enthält das Elektrolytsystem, in das der Implantatkörper eingetaucht wird, mindestens ein Phosphat. Insbesondere dann, wenn der Implantatkörper Eisen enthält, reichert sich die Oberfläche des Implantatkörpers aufgrund des Vorhandenseins von Phosphat in dem Elektrolyt mit biokompatiblen Eisenphosphatverbindungen an. Da die biokompatiblen Eisenphosphatverbindungen auf der Oberfläche an- und in die oberflächennahen Bereiche des Implantats eingelagert werden, werden Zellirritationen vermieden oder verringert. Der direkte Kontakt des vorzugsweise eisenhaltigen Materials des Implantatkörpers mit dem umgebenden Zellgewebe wird somit auf Zeiträume verschoben, die von dem Zeitpunkt weiter entfernt sind als bei herkömmlichen Implantaten.

Es ist weiterhin von Vorteil, dass nach der Einlagerung des Wasserstoffs eine Beschichtung des Implantatkörpers zumindest auf einem Teil seiner Oberfläche mit Magnesiumstearat und/oder Parylene und/oder einer pharmazeutisch aktiven Substanz, letztere insbesondere eingebettet in ein Polymer, beispielsweise ein Polylactid, ein Polyglycosid oder ein Copolymer aus diesen, besonders bevorzugt PLLA oder PLGA, oder ein Blend aus den genannten Polymeren erfolgt.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen.

Insbesondere vorteilhaft ist eine Beschichtung mittels eines Polymers, beispielsweise ein Polylactid, ein Polyglycosid oder ein Copolymer aus diesen, besonders bevorzugt PLLA oder PLGA, oder ein Blend aus den genannten Polymeren, welches die pharmazeutisch aktive Substanz enthält, da so die bei der Degradation des Polymers erzeugte Verringerung des pH-Werts im Bereich der Implantatoberfläche einen zusätzlichen Beschleunigungsfaktor für die Korrosion, insbesondere bei einem Implantat mit einer Eisenlegierung, darstellt.

Eine Beschichtung der Oberfläche des Implantats nach der Einlagerung von Wasserstoff mittels Parylene und/oder Magnesiumstearat ist von Vorteil, da die Oberflächeneigenschaften des Implantats nach der Wasserstoffeinlagerung durch die darüber liegende Beschichtung gewissermaßen 'eingefroren' werden. Hierdurch können die Oberflächeneigenschaften, die sonst ggf. von der Lagerungs- oder Transportdauer des Implantats bis zum Einbringen in den zu behandelnden Organismus abhängen, und damit auch die Degradationsdauer reproduzierbar und definiert eingestellt werden.

Vorteilhaft wirkt sich bei einer Beschichtung mit Parylene die hohe Spaltgängigkeit von Parylene aus, so dass auch eine tiefgehende Penetration der durch die Wasserstoff-Behandlung erzeugten rauen Oberflächen bis hin zu deren Gründen erfolgt. Die für Parylene charakteristischen Permeationseigenschaften für Wasser, chloridhaltige Lösungen und Wasserstoff sorgen in Verbindung mit der darunter liegenden wasserstoffbeladenen oberflächennahen Grenzschicht für ein besonders gut steuerbares Degradationsverhalten des Implantats. Dieses zeichnet sich auch durch einen über den Implantatquerschnitt uniformen langsamen Korrosionsfortschritt aus.

Parylene ist ein vollständig lineares, teilkristallines, unvernetztes aromatisches Polymer. Die verschiedenen Polymere besitzen unterschiedliche Eigenschaften und lassen sich in vier Grundtypen einteilen, nämlich Parylene C, Parylene D, Parylene N und Parylene F. Für die Anwendung als weitere Beschichtung nach einer Wasserstoffbeladung wird vorzugsweise Parylene C verwendet.

Mittels des erfindungsgemäßen Verfahrens kann bei der Beschichtung mit Magnesiumstearat ein Implantat hergestellt werden, das sich durch Defektfreiheit der Körperoberfläche durch nachträgliches Versiegeln auszeichnet. Lokale Fehlstellen und/oder auf der Körperoberfläche des Implantats vorhandene Poren und aufgeraute Bereiche werden wirksam vor dem Kontakt mit korrosiv wirkenden Körperflüssigkeiten geschützt. Die hydrophobe Oberflächeneigenschaft und der geringe Kristallwassergehalt des Magnesiumstearats, der auch durch einen vorzugsweise durchgeführten, sich an das Aufbringen der Magnesiumstearat-Beschichtung anschließenden Trocknungsschritt erzeugt wird, bewirken während der sich anschließenden Lagerung und dem Transport des Implantats eine äußerst geringe Diffusion von Wasser zum Grundwerkstoff des Implantatkörpers. Sowohl die an der Oberfläche des Implantats vorhandenen, herstellungsbedingten lokalen Kontaminationen als auch die durch die Legierungszusammensetzung des Implantat-Körpers an der Oberfläche liegenden Ausscheidungen werden durch das Magnesiumstearat inert eingebettet und können somit nicht mehr mit den Umgebungsbedingungen reagieren. Ebenso wird das Loslösen von Partikeln mit geringer Bindungsneigung von der Oberfläche des Implantat-Körpers beim Dilatieren verhindert. Diese Partikel verbleiben in der zähen, hoch flexiblen Magnesiumstearatschicht. Hierdurch ergibt sich eine erhöhte Hämo- beziehungsweise Biokompatibilität.

Aufgrund der Magnesiumstearat-Beschichtung des Implantat-Körpers wird in vorteilhafter Weise bewirkt, dass der Reibungskoeffizient des Implantats sinkt. Hieraus folgt, dass beim Verschieben beispielsweise eines Stents als Implantat in einem Katheter geringere Kräfte aufgewendet werden müssen. Dadurch wird im Falle eines Stents eine genauere Stentfixation ermöglicht. Zudem werden das Crimpen und die spätere Freisetzung des Implantats an dem zu behandelnden Ort vereinfacht.

In einem bevorzugten Ausführungsbeispiel des erfindungsgemäßen Verfahrens wird die Magnesiumstearat-Beschichtung mittels Eintauchen in eine Lösung aufgebracht, wobei die Lösung Magnesiumstearat und ein Lösungsmittel, vorzugsweise Aceton und/oder Isopropanol, enthält und vorzugsweise eine Temperatur zwischen etwa 10°C und 40°C aufweist. Alternativ kann die Magnesiumstearatschicht auch derart aufgebracht werden, dass die genannte magnesiumstearathaltige Lösung auf den Körper des Implantats aufgesprüht wird (spray coating). Dabei wird das Teil in einer Kammer an einem dünnen Draht aufgehängt und mittels einem rotierenden Teller (Chargenhalter) allseitig besprüht.

In einem bevorzugten Ausführungsbeispiel kann die Effektivität des Tauchprozesses durch Anlegen eines Drucks erfolgen, der kleiner als der Umgebungsdruck, vorzugsweise kleiner als etwa 90% des Umgebungsdrucks ist, d.h. des Luftdrucks an dem Ort, an dem der Tauchprozess durchgeführt wird. Der hierbei eintretende Entgasungseffekt führt zu einer schnellen Füllung der filigranen Oberflächenstruktur des Implantats mit Magnesiumstearat. Nach einer Verweildauer von einigen Minuten in der Lösung, vorzugsweise von mindestens etwa 2 Minuten, wird der mit Magnesiumstearat beschichtete ImplantatKörper aus dem Tauchbad entfernt und im Trockenofen bei einer Temperatur, die größer ist als die Raumtemperatur, vorzugsweise größer als etwa 30°C, getrocknet. Hierbei ist es besonders bevorzugt, wenn die Trocknungstemperatur möglichst gering ist, d.h. zwischen etwa 40°C und etwa 70°C liegt, da hierdurch ein langsames Freisetzen/Abdampfen des mindestens einen Lösungsmittels führt, wodurch eine porenfreie erste Schicht, die Magnesiumstearat enthält, erzeugt wird.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf. Die durch die Einlagerung von Wasserstoff entstehenden Oberflächenmorphologien und Oberflächenzusammensetzungen sind charakteristisch für diese Behandlung und am fertig hergestellten Implantat erkennbar.

Ferner wird die obige Aufgabenstellung durch ein biodegradierbares Implantat mit einem Körper, der überwiegend Eisen aufweist, gelöst, wobei in zumindest einen Teil des oberflächennahen Gefüges des Implantatkörpers Wasserstoff in einer Konzentration von etwa 50 ppm bis etwa 150 ppm eingelagert ist, wobei die Grenzschicht eine Dicke von maximal etwa 15 µm aufweist. Die angegebene Dicke der Grenzschicht mit Wasserstoff ist hinsichtlich Degradationsverhalten einerseits und Sprödbruchgefahr andererseits optimal. Wie oben bereits erläutert wurde, führt eine Konzentration von Wasserstoff in dem angegebenen Konzentrationsbereich zu einer besonders effektiven Beschleunigung der Degradation.

Es ist weiter bevorzugt, dass das Implantat eine aufgeraute Oberfläche aufweist. Durch die oben beschriebenen Einlagerungsprozesse von Wasserstoff wird eine erhöhte Rauhigkeit der Oberfläche des Implantatkörpers erzielt, welche für die Aufbringung einer weiteren Deckschicht (bspw. Magnesiumstearat) oder das Degradationsverhalten vorteilhaft ist.

Wie bereits oben erläutert, ist es ferner vorteilhaft, wenn der Implantatkörper in der oberflächennah angeordneten Grenzschicht ein Phosphat aufweist. Hierdurch wird insbesondere bei Verwendung einer Eisenlegierung für den Implantatkörper die Biokompatibilität des Implantats zumindest zu Beginn der Degradation verbessert.

Ebenfalls ist bevorzugt, wenn die Oberfläche des Implantatkörpers zumindest teilweise eine Beschichtung aufweist, welche Magnesiumstearat und/oder Parylene und/oder eine pharmazeutisch aktive Substanz enthält. Hierbei liegen bevorzugte Schichtdicken der Parylene-Beschichtung zwischen etwa 0,5 µm und etwa 10 µm.

Die bevorzugte Dicke der Magnesiumstearat-Beschichtung liegt bei etwa 0,5 µm bis etwa 10 µm, vorzugsweise etwa 1,0 µm bis etwa 5,0 µm. Die Konzentration des Magnesiumstearats in der weiteren Beschichtung liegt dabei etwa zwischen 80 Gew.% und 100 Gew.%.

Durch die Einlagerung von Wasserstoff in eine oberflächennahe Grenzschicht und die anschließende weitere Beschichtung mittels Magnesiumstearat und/oder Parylene kann die Degradationszeit des Implantats in weiten Grenzen entsprechend des jeweiligen Einsatzzwecks des Implantats variiert und definiert eingestellt werden.

Der Körper des Implantats enthält überwiegend Eisen, insbesondere mehr als 80 Gew.% Eisen, besonders bevorzugt mindestens 99 Gew.% Eisen, insbesondere in einer Legierung. Als weitere metallische Materialien können alternativ oder zusätzlich Mangan, Zink und/oder Wolfram eingesetzt werden. Diese Implantate werden, da sie kostengünstig herstellbar sind, besonders gern für die Behandlung von Erkrankungen des menschlichen oder tierischen Organismus eingesetzt. Insbesondere bei eisenhaltigen Implantaten führt die Einlagerung von Wasserstoff zu einer verringerten Degradationsdauer. Hierdurch wird eine Lücke zwischen den degradierbaren und nicht degradierbaren Legierungen für Implantate geschlossen.

Das erfindungsgemäße Verfahren bzw. das erfindungsgemäße Implantat wird nachfolgend in Beispielen erläutert. Dabei bilden alle beschriebenen Merkmale den Gegenstand der Erfindung, unabhängig von ihrer Zusammenfassung in den Ansprüchen oder deren Rückbezüge.

### 1. Vergleichsbeispiel:

Ein mittels Laserschneiden, Entgraten und Elektropolieren hergestelltes Implantat in Form eines Stents, der aus einer Eisenbasislegierung besteht, wird in 30 %iger HCl zehn Minuten bei Raumtemperatur gebeizt und anschließend in destilliertem Wasser gespült. Dabei erleidet der Stent je nach Legierungszusammensetzung einen Masseabtrag von 3 bis 8%. Die Rauheit der Stentoberfläche steigt. Es ist eine Erhöhung des integralen Wasserstoffgehaltes in einer Grenzschicht von 15 ppm auf 30 ppm zu verzeichnen. Der Wasserstoff kann im Trägergas - Heißextraktions - Verfahren nachgewiesen werden. Die angegebene Wasserstoff-Konzentration in der Grenzschicht liegt noch nicht im oben angegebenen optimalen Konzentrationsbereich, es wird jedoch bereits bei der angegebenen Wasserstoffkonzentration eine Beschleunigung der Degradation erreicht, insbesondere dann, wenn der behandelte Teil der Grenzschicht einen erhöhten Umformgrad aufweist.

### 1. Beispiel:

Ein analog zum ersten Vergleichsbeispiel vorbereiteter Stent aus einer Eisenbasislegierung wird zehn Minuten in einer 20 %igen HNO₃ bei Raumtemperatur gebeizt und danach in destilliertem Wasser gespült. Daran schließt sich eine kathodische Behandlung in einer alkalischen Phosphatlösung an, die mindestens eine Verbindung der Gruppe Natriumphosphat, Kaliumphospat, Kalziumdihydrogenphosphat, Dinatriumphosphat, Dikaliumphosphat und Kalziumhydrogenphosphat enthält. Hierbei sind Dinatriumphosphat, Dikaliumphosphat und Kalziumhydrogenphosphat weniger wasserlöslich als die übrigen Verbindungen der Gruppe.

Beispielsweise wird ein Stent in einer wässrigen Lösung mit 80g/l KH₂PO₄ mit einem Edelstahldraht kontaktiert und anodisch geschaltet. Der pH-Wert der Lösung beträgt dabei ca. 9. Bei einer Badspannung zwischen 2 und 8 V und einer Stromdichte von 0,5 bis 1,5 A/dm² erfolgt über einen Zeitraum von 1 bis 5 min bei Raumtemperatur eine Beladung der Stentoberfläche mit negativ geladenen Phosphationen. Diese bilden an der Stentoberfläche eine dünne Schicht aus schwer wasserlöslichen Eisenphosphaten (Fe(II)- oder Fe-(III)-phosphaten. Ebenso ist die Bildung von Eisendiphosphat möglich. Die maximale Schichtdicke beträgt dabei etwa 0,5 µm. Es ist darauf zu achten, dass keine höhere Stromdichte und keine Erhöhung der Behandlungszeit zugelassen wird, da sonst der Wasserstoffgehalt in der Randschicht zu sehr minimiert werden würde.

Die nunmehr aus Fe-phosphaten bestehende Oberfläche weist eine höhere Biokompatibilität als die reine Eisenoberfläche auf. Zudem bewirkt diese Oberfläche einen temporären Oberflächenschutz. Dies bedeutet, dass der Eisenstent während seiner Lagerzeit vorerst nicht weiter korrodiert und somit keine Verkürzung der Mindesthaltbarkeit des Katheters erforderlich wird. Die Eisenphosphatoberfläche ist auf der anderen Seite aber nicht korrosionsstabil genug, um dem korrosiven Angriff im Blutgefäß einen größeren Widerstand entgegen zu setzen. Damit fungiert die eisenphosphathaltige Oberfläche als temporär (über die Lagerzeit) wirkender Inhibitor mit erhöhter Biokompatibilität.

### 2. Beispiel:

Analog zum 1. Beispiel, wobei jedoch die kathodische Behandlung des Implantats in einer sauren, phosphorsäurehaltigen Lösung erfolgt. In der phosphorsäurehaltigen Lösung sind bspw. die in Beispiel 2 genannten Phosphate gelöst.

Die Lösung besteht insbesondere aus einer wässrigen 10 -30%-igen Phosphorsäure. In dieser wird der Eisenstent mit einem Edelstahldraht kontaktiert und analog zu Beispiel 1 anodisch unter Verwendung der identischen Strom- und Spannungsparameter geschaltet. Dabei bewirkt das saure Milieu eine weitere Zerklüftung der Stentoberfläche. Es bilden sich ebenfalls verschiedene Eisenphosphate.

### 3. Beispiel:

Analog zu Beispiel 1 bis 2 mit zusätzlicher Versiegelung der rauen Oberfläche des Implantatkörpers mit Magnesiumstearat oder Parylene C.

Die Beschichtung mit Parylene C erfolgt aus der Gasphase. Nach ca. einer halben Stunde Beschichtungszeit wird eine Schichtdicke von ca. 0,5 µm erzielt.

Alternativ wird eine Magnesiumstearatbeschichtung auf die Implantatoberfläche aufgetragen. Nach der Durchführung einer der Ausführungsbeispiele 1 bis 2 und einer daran anschließenden Trocknung, wird die Endoprothese auf einen Kunststofffaden (z.B. Polyamid) aufgehängt und anschließend in die Lösung zur Auftragung des Magnesiumstearats getaucht. Die Lösung besteht z.B. aus 9 Teilen hochreinem Aceton oder Isopropanol und 1 Teil Magnesiumstearat. Der Tauchvorgang erfolgt bei Raumtemperatur in einem evakuierbaren Exsikkator. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Stentoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt dabei im Bereich von etwa 0,5 bis etwa 10 µm.

Bedingt durch den im Exsikkator vorliegenden Unterdruck wird das Magnesiumstearat sehr gleichmäßig auf der Oberfläche abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht entsteht. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

Das Magnesiumstearat bewirkt einen zusätzlichen Versiegelungseffekt der Implantatoberfläche. Das bedeutet, dass ein so behandeltes Implantat entweder länger (z.B. bis zur Stentmontage in das Kathetersystem) gelagert werden kann, oder im Falle eines orthopädischen Implantates eine höhere Standzeit/ Funktionsfähigkeit aufweist. Damit ergibt sich die Möglichkeit bis zur Resorption des Magnesiumstearates eine längere mechanische Stabilität aufrecht zu erhalten. Dies eröffnet z.B. die Anwendung für einen absorbierbaren Marknagel in der Orthopädie. Dieser wird nach einigen Monaten, wenn seine Stützwirkung langsam nicht mehr vonnöten ist, von den Osteoklasten der Spongiosa resorbiert. Die Magnesiumstearat-Behandlung bietet zudem den Vorteil, dass das aufwändige Elektropolieren des Implantats (insbesondere bei Stents) entfallen oder mit einem wesentlich geringeren Aufwand betrieben werden kann.

Analog zu der Herstellung der Parylene- oder Magnesiumstearat-Beschichtung kann die zerklüftete Oberfläche des Implantats alternativ oder zusätzlich mit einer pharmazeutisch aktiven Substanz beschichtet werden. Bevorzugte Substanzen sind oben in der Beschreibung angegeben.

Der Nachweis der erhöhten Degradation von Implantaten, welche mit Wasserstoff beladen sind, kann bspw. durch Auslagerung in PBS (Phosphate buffered solution) oder in SBF (simulated body fluid) erfolgen. Bei entsprechend behandelten Stents mit einem Körper aus einer Eisenlegierung mit einer Zusammensetzung von >50 Gew.% bis 99,99 Gew.% Eisen mit Legierungselementen wie Mn, Si, Pd, Pt, N, C, S und ggf. weiteren Legierungsbestandteilen konnte nach Auslagerung in SBF eine Erhöhung der Fe-Elution bis zu einem Faktor von 1,5 festgestellt werden. Nach der Auslagerung in PBS wurde zudem durch den Aufrauungseffekt eine Vergrößerung der realen Oberfläche um den Faktor 1,5 erreicht.

Bei der Dimensionierung der erfindungsgemäßen Wasserstoff-Beladung des Implantatkörpers müssen auch die jeweiligen Abmessungen des Implantats berücksichtigt werden. Dies soll im Folgenden beispielhaft anhand eines Stents gezeigt werden.

Insgesamt sollte der Durchmesser des nicht mit einem erhöhten Wasserstoffgehalt beladenen inneren Gefügebereichs eines Stentstegs mindestens 50% des Gesamtdurchmessers des Stentstegs betragen. Andernfalls besteht die Gefahr, dass die von der Randzone loslaufenden Risse nicht mehr in der Bauteilmitte gestoppt werden können. In diesem Fall kann der jeweilige Steg seiner Stützfunktion nicht mehr nachkommen.

Es kann abgeschätzt werden, dass bei einer Dicke der Grenzschicht mit erhöhter Wasserstoffkonzentration von 5 µm Risse bis in eine Tiefe von 10 µm in die Matrix hinein laufen und die Standzeit des Stegs eines 100 µm breiten Stents dadurch auf 80% sinkt. Bei einer Grenzschichtdicke von 15 µm werden die Risse durch das Gefüge des Stentkörpers nach ca. 25 µm aufgehalten. Somit muss bei einer Grenzschichtdicke von 15 µm der Stegquerschnitt eines Stents oder eines anderen Implantates mindestens 170 µm x 170 µm betragen (= 0,289 mm²). Die verbleibende rissfreie Querschnittsfläche würde dann Kantenlängen von 120 µm x 120 µm (=0,144 mm²) haben und eine Degradationsdauer im angestrebten Zeitbereich gewährleisten.

## Patentansprüche

1. Verfahren zur Herstellung eines biodegradierbaren Implantats mit einem Körper, der überwiegend Eisen aufweist umfassend die folgenden Schritte:
a) Bereitstellen des Körpers des Implantats,
b) Einlagerung von Wasserstoff in zumindest einen Teil des oberflächennahen Gefüges des Implantatkörpers durch Beizen mittels einer Säure, wobei im Anschluss an den Beizschritt zur Einlagerung des Wasserstoffs zumindest ein Teil des Implantatkörpers in ein saures bis basisches Elektrolytsystem getaucht und anschließend mit der Kathode einer Spannungsquelle verbunden wird.

2. Verfahren nach Anspruch 1, **dadurch gekennzeichnet, dass** die Einlagerung von Wasserstoff in das Gefüge einer oberflächennah angeordneten Grenzschicht des Implantatkörpers erfolgt, wobei die Grenzschicht eine Dicke von maximal 15 µm aufweist.

3. Verfahren nach Anspruch 1 oder 2, **dadurch gekennzeichnet, dass** die mittlere Konzentration von Wasserstoff in den Gefügebereichen des Implantatkörpers, in denen die Einlagerung erfolgt, nach Abschluss der Einlagerung 50 ppm bis 150 ppm beträgt.

4. Verfahren nach einem der vorhergehenden Ansprüche, **dadurch gekennzeichnet, dass** der Körper des Implantats anschließend an die Einlagerung des Wasserstoffs in destilliertem Wasser gespült wird.

5. Verfahren nach einem der vorhergehenden Ansprüche, **dadurch gekennzeichnet, dass** zur Einlagerung des Wasserstoffs zumindest ein Teil des Implantatkörpers in ein saures bis basisches Elektrolytsystem getaucht und anschließend mit der Kathode einer Spannungsquelle verbunden wird, die mit einer Stromdichte von 0,5 A/dm² bis 2 A/dm² beaufschlagt wird.

6. Verfahren nach Anspruch 5, **dadurch gekennzeichnet, dass** das Elektrolytsystem mindestens ein Phosphat enthält.

7. Verfahren nach einem der vorhergehenden Ansprüche, **dadurch gekennzeichnet, dass** nach der Einlagerung des Wasserstoffs eine Beschichtung des Implantatkörpers zumindest auf einem Teil seiner Oberfläche mit Magnesiumstearat und/oder Parylene und/oder einer pharmazeutisch aktiven Substanz erfolgt.

8. Biodegradierbares Implantat mit einem Körper, der überwiegend Eisen aufweist, wobei in zumindest einen Teil des oberflächennahen Gefüges des Implantatkörpers Wasserstoff eingelagert ist, wobei der Wasserstoff in das Gefüge einer oberflächennah angeordneten Grenzschicht des Implantatkörpers eingelagert ist und die Grenzschicht eine Dicke von maximal 15 µm aufweist, **dadurch gekennzeichnet, dass** in zumindest einen Teil des oberflächennahen Gefüges des Implantatkörpers Wasserstoff in einer Konzentration von 50 ppm bis 150 ppm eingelagert ist.

9. Implantat nach Anspruch 8, **dadurch gekennzeichnet, dass** die Oberfläche des Implantatkörpers zumindest teilweise eine Beschichtung aufweist, welche Magnesiumstearat und/oder Parylene und/oder eine pharmazeutisch aktive Substanz enthält.

10. Implantat nach einem der Ansprüche 8 oder 9, **dadurch gekennzeichnet, dass** der Implantatkörper in der oberflächennah angeordneten Grenzschicht ein Phosphat aufweist.

11. Implantat nach einem der Ansprüche 8 bis 10, **dadurch gekennzeichnet, dass** der Körper des Implantats mehr als 99 Gew.% Eisen, insbesondere in einer Legierung, enthält.

## Claims

1. A method for producing a biodegradable implant having a body containing predominantly iron, comprising the following steps:
a) providing the body of the implant,
b) incorporating hydrogen into at least a portion of the structure of the implant body near the surface by pickling by means of an acid, wherein following the pickling step for incorporating the hydrogen at least a portion of the implant body is immersed in an acidic to alkaline electrolyte system and is then connected to the cathode of a voltage source.

2. The method according to claim 1, **characterised in that** the hydrogen is incorporated into the structure of a boundary layer of the implant body near the surface, wherein the boundary layer has a thickness of at most 15 µm.

3. The method according to claim 1 or 2, **characterised in that** the average concentration of hydrogen in the structural areas of the implant body where the incorporation of hydrogen takes place is 50 ppm to 150 ppm after completion of the incorporation.

4. The method according to any one of the preceding claims, **characterised in that** the body of the implant is rinsed in distilled water subsequently to the incorporation of the hydrogen.

5. The method according to any one of the preceding claims, **characterised in that**, to incorporate the hydrogen, at least a portion of the implant body is immersed in an acidic to alkaline electrolyte system for the incorporation of hydrogen, and then is connected to the cathode of a voltage source, which is preferably acted upon with a current density of from 0.5 A/dm² to 2 A/dm².

6. The method according to claim 5, **characterised in that** the electrolyte system contains at least one phosphate.

7. The method according to any one of the preceding claims, **characterised in that**, following the incorporation of the hydrogen, the implant body is coated at least over a portion of its surface with magnesium stearate and/or parylene and/or a pharmaceutically active substance.

8. A biodegradable implant having a body containing predominantly iron, wherein hydrogen is incorporated into at least a portion of the structure of the implant body near the surface, wherein the hydrogen is incorporated into the structure of a boundary layer of the implant body near the surface and the boundary layer has a thickness of at most 15 µm, **characterised in that** hydrogen is incorporated in a concentration of from 50 ppm to 150 ppm in at least a portion of the structure of the implant body near the surface.

9. The implant according to claim 8, **characterised in that** the surface of the implant body at least partially has a coating containing magnesium stearate and/or parylene and/or a pharmaceutically active substance.

10. The implant according to either one of claims 8 or 9, **characterised in that** the implant body comprises a phosphate in the boundary layer arranged near the surface.

11. The implant according to any one of claims 8 to 10, **characterised in that** the body of the implant contains more than 99 % by weight iron, in particular in an alloy.

## Revendications

1. Procédé de fabrication d'un implant biodégradable doté d'un corps qui présente principalement du fer, comprenant les étapes suivantes :
a) la préparation du corps de l'implant,
b) l'incorporation d'hydrogène dans au moins une partie de la structure proche de la surface du corps d'implant par un décapage au moyen d'un acide, où, à l'issue de l'étape de décapage, au moins une partie du corps d'implant est immergée dans un système d'électrolyte acide à basique pour l'incorporation de l'hydrogène, et ensuite est reliée avec la cathode d'une source de tension.

2. Procédé selon la revendication 1, **caractérisé en ce que** l'incorporation d'hydrogène a lieu dans la structure d'une couche limitrophe du corps d'implant disposée proche de la surface, où la couche limitrophe présente une épaisseur d'au maximum 15 µm.

3. Procédé selon la revendication 1 ou la revendication 2, **caractérisé en ce que** la concentration moyenne d'hydrogène dans les zones de structure du corps d'implant dans lesquelles a lieu l'incorporation, est de 50 ppm à 150 ppm à la fin de l'incorporation.

4. Procédé selon l'une des revendications précédentes, **caractérisé en ce que** le corps de l'implant est lavé dans de l'eau distillée à la suite de l'incorporation d'hydrogène.

5. Procédé selon l'une des revendications précédentes, **caractérisé en ce que**, pour l'incorporation d'hydrogène, au moins une partie du corps d'implant est plongée dans un système d'électrolyte acide à basique et est ensuite reliée avec la cathode d'une source de tension qui est soumise à une densité de courant de 0,5 A/dm² à 2 A/dm².

6. Procédé selon la revendication 5, **caractérisé en ce que** le système électrolyte contient au moins un phosphate.

7. Procédé selon l'une des revendications précédentes, **caractérisé en ce qu'**après l'incorporation de l'hydrogène, il est procédé à un revêtement du corps d'implant, au moins sur une partie de sa surface, avec du stéarate de magnésium et/ou du parylène et/ou une substance pharmaceutiquement active.

8. Implant biodégradable doté d'un corps, qui présente principalement du fer, où de l'hydrogène est incorporé dans au moins une partie de la structure du corps d'implant proche de la surface, où l'hydrogène est incorporé dans la structure d'une couche limitrophe du corps d'implant disposée proche de la surface et la couche limitrophe présente un épaisseur d'au maximum 15 µm, **caractérisé en ce que** de l'hydrogène dans une concentration de 50 ppm à 150 ppm est incorporé dans au moins une partie de la structure du corps d'implant proche de la surface.

9. Implant selon la revendication 8, **caractérisé en ce que** la surface du corps d'implant présente au moins partiellement un revêtement, lequel contient du stéarate de magnésium, et/ou du parylène, et/ou une substance pharmaceutiquement active.

10. Implant selon la revendication 8 ou la revendication 9, **caractérisé en ce que** le corps d'implant présente un phosphate dans la couche limitrophe disposée proche de la surface.

11. Implant selon l'une des revendications 8 à 10, **caractérisé en ce que** le corps de l'implant contient plus de 99 % en poids de fer, notamment dans un alliage.
